(19) Europäisches Patentamt
European Patent Office
Office européen des brevets

(11) Veröffentlichungsnummer: **0 115 328**
**B1**

(12) **EUROPÄISCHE PATENTSCHRIFT**

(45) Veröffentlichungstag der Patentschrift: **19.02.86**

(51) Int. Cl.⁴: **C 07 C 143/70**, C 07 C 143/78, C 07 C 143/825, C 07 C 143/68, C 07 C 145/00, C 07 C 147/06

(21) Anmeldenummer: **84100731.3**

(22) Anmeldetag: **25.01.84**

(54) **Verfahren zur Herstellung von 4-Chlorphenylsulfonylverbindungen.**

(30) Priorität: **27.01.83 DE 3302647**

(43) Veröffentlichungstag der Anmeldung: **08.08.84 Patentblatt 84/32**

(45) Bekanntmachung des Hinweises auf die Patenterteilung: **19.02.86 Patentblatt 86/8**

(84) Benannte Vertragsstaaten: **CH DE FR GB IT LI**

(56) Entgegenhaltungen:
**EP - A - 0 020 800**
**EP - A - 0 062 736**
**EP - B - 0 001 275**
**CH - A - 634 046**
**DE - A - 2 635 279**
**DE - A - 2 721 429**
**DE - A - 3 140 854**
**US - A - 3 772 373**

**DR. OTTO-ALBRECHT NEUMÜLLER, "Römpps Chemie-Lexikon", 7. Auflage, Band 5, 1975 FRANCKH'SCHE VERLAGSHANDLUNG, STUTTGART SEITE 3397**
**ULLMANNS ENCYKLOPÄDIE DER TECHNISCHEN CHEMIE, 4. Auflage, Band 8, 1974 VERLAG CHEMIE, Weinheim/Bergstrasse Seite 420**

(73) Patentinhaber: **HOECHST AKTIENGESELLSCHAFT, Postfach 80 03 20, D-6230 Frankfurt am Main 80 (DE)**

(72) Erfinder: **Papenfuhs, Theodor, Dr., Heinrich-Bleicher-Strasse 40, D-6000 Frankfurt am Main 50 (DE)**

**EP 0 115 328 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist ein abwasserfreies und damit ökologisch optimales Verfahren zur Herstellung von 4-Chlorphenylsulfonylverbindungen der allgemeinen Formel (1)

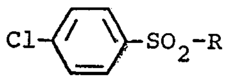 (1)

in welcher R ein Wasserstoff- oder Chloratom oder eine Alkyl-, Oxalkyl- oder Alkoxygruppe, eine Hydrazinogruppe, eine primäre Aminogruppe, primäre oder sekundäre Alkylaminogruppe, Aralkylaminogruppe oder Phenylaminogruppe bedeutet, wobei die Phenylgruppe durch Chlor- oder Bromatome, Alkylgruppen, Alkoxygruppen oder Phenoxygruppen substituiert sein kann, oder eine 4-Chlorphenylgruppe bedeutet, durch Reaktion von Chlorbenzol mit Chlorsulfonsäure und Thionylchlorid zum 4-Chlorbenzolsulfochlorid und dessen Umsetzung mit Ammoniak, Hydrazin, primärem oder sekundärem aliphatischen Amin, aromatischem Amin, Alkalimetallsulfit oder Alkanol zu Verbindungen der genannten Formel (1), dadurch gekennzeichnet, dass man 1 Mol Chlorbenzol langsam mit einem Gemisch aus 1,0 bis 1,05 Mol Chlorsulfonsäure und 1,0 bis 1,25 Mol Thionylchlorid bei Temperaturen von 20 bis 90°C mit der Massgabe, dass das Reaktionsgemisch stets flüssig ist, ggf. in Gegenwart eines für die Sulfonsäurechloridbildung mittels Thionylchlorid an sich bekannten Katalysators, umsetzt und das hierbei in Form einer Schmelze angefallene 4-Chlorbenzolsulfochlorid vorzugsweise direkt oder nach Abtrennung von dem als Nebenprodukt angefallenen 4,4'-Dichlordiphenylsulfon umsetzt

a) mit einer wässerigen Lösung, Suspension oder Emulsion von Ammoniak, Hydrazin, eines primären oder sekundären aliphatischen Amins oder aromatischen Amins der Benzolreihe bei Temperaturen von 0 bis 100°C zu den entsprechenden 4-Chlorbenzolsulfonamiden, oder

b) mit einer wässerigen Alkalimetallsulfitlösung bei Temperaturen von 20 bis 90°C bei pH-Werten von 6 bis 9 zu den entsprechenden 4-Chlorbenzolsulfinaten und daraus durch Neutralisieren der wässerigen Lösung mittels Säure zu der freien 4-Chlorbenzolsulfinsäure oder durch Umsetzen mit Alkylierungsmitteln zu den entsprechenden 4-Chlorphenylalkylsulfonen, oder

c) mit der mindestens stöchiometrischen Menge eines Alkanols bei Temperaturen von 20 bis 90°C zu den entsprechenden 4-Chlorbenzolsulfonsäurealkylestern, oder

d) mit der mindestens stöchiometrischen Menge Chlorbenzol in Gegenwart katalytischer Menge Eisen(III)-chlorid bei Temperaturen von 120-160°C zum 4,4'-Dichlordiphenylsulfon.

Sofern bei der Definition von R der allgemeinen Formel (1) Alkyl-, Oxyalkyl- oder Alkoxygruppen genannt werden, handelt es sich vorzugsweise um solche von 1-6 Kohlenstoffatomen. Ebenso sind unter den in den vorstehend auftretenden Begriffen «aliphatisches Amin», «Alkylierungsmittel», «Alkylsulfonen», «Alkanol» und «Sulfonsäurealkylester» enthaltenen Alkyl- bzw. -aliphatischen Resten jeweils vorzugsweise solche von 1 bis 6 Kohlenstoffatomen zu verstehen.

Durch das erfindungsgemäss Verfahren wird es ermöglicht, die Verbindungen der genannten Formel (1), ausgehend vom Chlorbenzol, ohne Zwischenisolierung des zunächst erhaltenen 4-Chlorbenzolsulfochlorids in einer Eintopfreaktion herzustellen, wobei die in erster Stufe ablaufende Sulfochlorierung in abwasserfreier Form und damit ökologisch optimal erfolgt.

Der erste Verfahrensschritt betrifft die Überführung des Chlorbenzols in das 4-Chlorbenzolsulfochlorid durch Chlorsulfonierung. Diese Reaktion ist im Prinzip bekannt und wird technisch durch Umsetzung von Chlorbenzol mit überschüssiger Chlorsulfonsäure durchgeführt, wobei aber nach beendeter Reaktion aufgrund der notwendigen hydrolytischen Zersetzung des Reaktionsgemisches grosse Mengen schwefel- und salzsäurehaltiger Mutterlaugen anfallen, deren Entsorgung äusserst aufwendig, wegen der sonst unvermeidlichen Umweltbelastung jedoch unumgänglich ist. Es hat daher nicht an Versuchen gefehlt, die Chlorsulfonierung von Chlorbenzol mit reduzierten Mengen an Chlorsulfonsäure durchzuführen, um die Abwasserkosten zu senken. Zur Erzielung hoher Ausbeuten musste hierbei in einem nachgeschalteten Verfahrensschritt überschüssiges Thionylchlorid zum Umsetzungsgemisch aus Chlorbenzol und Chlorsulfonsäure gegeben werden. Im günstigsten Fall liess sich auf diese Weise die Chlorsulfonsäuremenge auf den stöchiometrischen Bedarf (1 Mol pro Mol Chlorbenzol) reduzieren. Allerdings war beim zweiten Verfahrensschritt ein erheblicher Thionylchlorid-Überschuss unvermeidbar (1,5-2,0 Mol pro Mol aromatischer Verbindung, gemäss Beispiel betreffend Chlorbenzol 2,0 Mol, der in einem dritten Verfahrensschritt destillativ wieder entfernt werden musste (Europäische Patentschrift 1275).

Bei dem in der DE-A1-2721429 beschriebenen Verfahren zur Herstellung von Benzolsulfonsäurechloriden wird das ggf. substituierte Benzol mit der etwa äquimolaren Menge eines Sulfonierungsmittels, bezogen auf jede einzuführende Sulfonsäurechloridgruppe, und einem Überschuss an Thionylchlorid von bis zu 10 Mol pro Benzolverbindung umgesetzt. Sofern als Sulfonierungsmittel Chlorsulfonsäure und als Benzolverbindung Chlorbenzol eingesetzt wird, wird nach dort gemachten Angaben in einem Beispiel 1 Mol Chlorbenzol mit 1,6 Mol Chlorsulfonsäure und 3,2 Mol Thionylchlorid, d.h. mit einem molaren Überschuss von 220% an Thionylchlorid umgesetzt.

Gegenüber den aus den beiden zitierten Literaturstellen bekannten Verfahren ist beim erfindungsgemässen Verfahren das Thionylchlorid ohne jeden Überschuss oder nur in einem geringen molaren Überschuss anwendbar.

Ausserdem war unter diesen Bedingungen, selbst unter Zusatz von Sulfonierungshilfsmitteln wie Dimethylformamid, Essig, oder Phosphorsäure, eine vollständige Umsetzung des Chlorbenzols nicht möglich, so dass der nicht umgesetzte Rest

des Ausgangsproduktes ebenfalls destillativ abzutrennen war. Da bei der beschriebenen bekannten Umsetzung von Chlorbenzol mit Chlorsulfonsäure, insbesondere mit einer stöchiometrischen Menge von Chlorsulfonsäure, und anschliessenden Umsetzung mit überschüssigem Thionylchlorid beträchtliche Mengen 4,4'-Dichlordiphenylsulfon als Nebenprodukt gebildet werden, muss zur Herstellung von technisch reinem 4-Chlorbenzolsulfochlorid eine Vakuumdestillation angeschlossen werden, die wegen der enthaltenen flüchtigen Ausgangsprodukte (Chlorbenzol/Thionylchlorid) als fraktionierte Destillation ausgelegt sein muss und daher erhöhten Investitionsaufwand erfordert.

Es ist als überraschend zu erachten, dass gemäss der ersten Stufe des erfindungsgemässen Verfahrens eine abwasserfreie Herstellung von 4-Chlorbenzolsulfochlorid bei nur geringem Anfall von 4,4'-Dichloridphenylsulfon und unter vollständigem Abreagieren des Ausgangsproduktes gelingt.

Die nach der Reaktionsgleichung

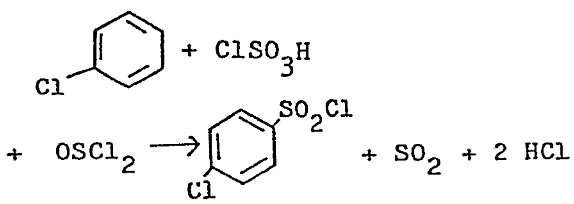


in stöchiometrischer Menge gebildeten Reaktionsgase (1 Mol $SO_2$ und 2 Mol HCl) können in einer zweistufigen, mit Wasser und anschliessend mit Alkalilauge betriebenen Abgaswäsche quantitativ zu wiederverwertbarer Salzsäure und Bisulfitlösung umgesetzt werden.

Man erhält nach Beendigung der ersten Reaktionsstufe eine Schmelze des p-Chlorbenzolsulfochlorids, die entweder durch einfache Vakuumdestillation, d.h. ohne eine aufwendige Fraktionierung, vom enthaltenen 4,4'-Dichlordiphenylsulfongetrennt und auf diese Weise in technisch reines 4-Chlorbenzolsulfochlorid in hoher Ausbeute überführt werden kann, oder, was besonders vorteilhaft ist, direkt für alle weiter obengenannten technisch wichtigen Folgereaktionen eingesetzt werden kann, wobei das erhaltene 4,4'-Dichlordiphenylsulfon im Zuge der Weiterverarbeitung, ggfs. durch Klärfiltration, abgetrennt und ebenfalls in reiner Form erhalten werden kann.

Gemäss dem Erfindungsgemässen Verfahren erhält man, ausgehend vom Chlorbenzol, ohne erforderliche Zwischenisolierung des zunächst gebildeten 4-Chlorbenzolsulfochlorids und ohne Verwendung von Lösungsmitteln bei minimalem Abwasseranfall, der ausschliesslich aus den genannten Folgestufen stammt, hohe Ausbeuten an 4-Chlorbenzol-sulfochlorid, -sulfonamiden, -sulfonsäureestern, -sulfinaten bzw. sulfinsäure, sowie 4-Chlorphenyl-alkylsulfonen.

Ferner werden durch die geschilderte Abgasbehandlung wiederverwendbare Salzsäure und Bisulfitlösung (beispielsweise für die Reduktion des 4-Chlorbenzolsulfochlorids und nachfolgende Neutralisation der wässerig-alkalischen Lösung im Zuge der Verfahrensvariante b) als Wertprodukte gewonnen.

Das erfindungsgemässe Verfahren wird im einzelnen wie folgt durchgeführt:

Man tropft zu einer Mischung aus zweckmässigerweise 1,0 bis 1,05 Mol Chlorsulfonsäure und 1,0-1,25 Mol Thionylchlorid, ggf. nach Versetzen mit 0,5-5 Molprozent eines Säurechlorierungs-Katalysators (wie beispielsweise Dimethylformamid, Dimethylacetamid, N-Methylpyrrolidon, N-Methylacetamid, Tetramethylharnstoff oder Triethylamin, wie sie in der Literatur, beispielsweise in Organikum, VEB Deutscher Verlag der Wissenschaften, 4. Auflage, Berlin 1964, oder in Helvetia Chimica Acta (1959), Band 42, Seite 1653 ff., vielfältig beschrieben sind) bei Temperaturen von 20 bis 90°C mit der Massgabe, dass das Reaktionsgemisch stets flüssig bleibt, vorzugsweise von 55-90°C, im Verlaufe von etwa 2 bis etwa 8 h, vorzugsweise 3-6 h, 1,0 Mol Chlorbenzol gleichmässig zu, wobei die entweichenden Reaktionsgase in einer nachgeschalteten zweistufigen Wäsche, die mit Wasser und Alkalilauge beschickt ist, zu Salzsäure und Bisulfitlösung umgesetzt werden.

Nach Beendigung der Gasentwicklung enthält die entstandene 4-Chlorbenzolsulfochlorid-Schmelze weder freie Chlorsulfonsäure oder Thionylchlorid noch nicht umgesetztes Chlorbenzol. (Die ggf. eingesetzten geringen Überschussmengen an Chlorsulfonsäure, maximal 0,05 Mol, und Thionylchlorid, maximal 0,25 Mol, werden mit den entweichenden Reaktionsgasen entsprechend ihrem bei Umsetzungstemperatur herrschenden Dampfdruck aus dem Umsetzungsgemisch entfernt. Sie können durch wenige Versuche für jede beabsichtigte Reaktionstemperatur experimentell ermittelt werden).

Vom zwangsweise gebildeten, allerdings gegenüber dem Stand der Technik vergleichsweise in nur geringer Menge angefallenen 4,4'-Dichlordiphenylsulfon (1-3 Molprozent) kann das 4-Chlorbenzolsulfochlorid anschliessend rein und in hoher Ausbeute abdestilliert werden. Besonders vorteilhaft ist es jedoch, wenn die erhaltene rohe 4-Chlorbenzolsulfochlorid-Schmelze direkt und ohne Reinigung zu den technisch genutzten, vorstehend beschriebenen Folgereaktionen a), b) u. c) eingesetzt wird, wobei das enthaltene 4,4'-Dichlordiphenylsulfon bei der Weiterverarbeitung abgetrennt wird.

Die Abtrennung kann durch Klärfiltration erfolgen, sofern eine wasser- oder alkalilösliche Zwischenstufe durchlaufen wird oder das Endprodukt alkali- oder wasserlöslich ist. Bei der Weiterverarbeitung der rohen 4-Chlorbenzolsulfochlorid-Schmelze zu destillierbaren Endprodukten erfolgt die Abtrennung zweckmässigerweise durch fraktionierte Destillation des Reaktionsgemisches.

Beispielsweise werden wasser- oder alkalilösliche 4-Chlorbenzol-sulfonamide (Vorprodukte für Pharmazeutika und Pigmente) hergestellt, indem man eine wässerige Lösung, Suspension oder Emulsion von Ammoniak, primären oder sekundärem aliphatischen oder ggf. kernsubstituiertem

aromatischem Amin bei Temperaturen zwischen 0 und 100°C, vorzugsweise zwischen 20 und 80°C, innerhalb von 1 bis 5 h mit der rohen 4-Chlorbenzolsulfochlorid-Schmelze versetzt und die gebildeten 4-Chlorbenzolsulfonamide, ggf. nach Zusatz stöchiometrischer Mengen an Alkalihydroxyd, bei geeigneter Temperatur in Form der freien wasserlöslichen Amide bzw. ihrer wasserlöslichen Alkalisalze in Lösung hält oder bringt, von ungelöstem 4,4'-Dichlordiphenylsulfon abfiltriert und die Zielprodukte durch Abkühlen, ggf. nach Zusatz mindestens äquivalenter Mengen Mineralsäure, abscheidet und durch Filtration isoliert.

Zur Herstellung der 4-Chlorbenzolsulfinate und der daraus erhältlichen 4-Chlorphenyl-alkylsulfone (Vorprodukte für Reaktivfarbstoffe) wird eine aus Alkalibisulfit (vorteilhaft aus der Abgasabsorptionsanlage der erfindungsgemässen 4-Chlorbenzolsulfochlorid-Synthese) und Alkalilauge hergestellte wässerige Alkalisulfitlösung bei 20 bis 90°C, vorzugsweise 40 bis 70°C, innerhalb von 1 bis 5 h, vorzugsweise 2 bis 4 h, gleichzeitig mit der rohen 4-Chlorbenzolsulfochlorid-Schmelze und Alkalilauge versetzt, wobei ein pH-Wert zwischen etwa 6 und 9 eingehalten wird. Die erhaltene wässerige Lösung des 4-Chlorbenzolsulfinats wird vom ungelösten 4,4'-Dichlordiphenylsulfon abfiltriert. Aus dem wässerigen Filtrat wird dann das 4-Chlorbenzolsulfinat durch Abkühlen und/oder Aussalzen, oder die freie 4-Chlorbenzolsulfinsäure durch Ansäuern in reiner Form abgeschieden und nachfolgend durch Filtration isoliert.

Alternativ kann das wässerige Filtrat (4-Chlorbenzolsulfinat) ohne Isolierung mit Alkylierungsmitteln (wie beispielsweise Dialkylsulfat, wie Dimethyl- oder Diäthylsulfat, Alkylhalogenid, wie Alkylchlorid, -bromid oder -jodid, oder Alkylenoxid, wie Äthylenoxid oder Propylenoxid) bei Temperaturen von 20 bis 150°C, vorzugsweise 50 bis 120°C, ggf. unter Druck, umgesetzt werden, wobei bei Verwendung von Alkylenoxid durch gleichzeitige Zugabe von Mineralsäure (vorzugsweise aus der Abgasabsorptionsanlage der erfindungsgemässen 4-Chlorbenzolsulfochlorid-Synthese) ein pH-Wert von 6 bis 8 eingehalten wird. Das gebildete 4-Chlorphenyl-alkylsulfon wird nach beendeter Umsetzung, ggf. nach Abkühlen und/oder Aussalzen durch Filtration oder Extraktion isoliert.

Zur Herstellung von 4-Chlorbenzolsulfonsäureestern wird rohe 4-Chlorbenzolsulfochlorid-Schmelze bei 20 bis 90°C, vorzugsweise 30 bis 60°C, zu einer mindestens stöchiometrischen Menge des umzusetzenden Alkohols innerhalb von 1 bis 5, vorzugsweise 2 bis 4 h getropft, bis zum Ende der sofort einsetzenden Gasentwicklung nachgerührt und der dabei gebildete 4-Chlorbenzolsulfonsäureester anschliessend im Vakuum abdestilliert. Das im Reaktionsgemisch enthaltene 4,4'-Dichlordiphenylsulfon verbleibt im Destillationsrückstand. Zur Herstellung der 4-Chlorbenzolsulfonsäureester wird vorzugsweise ein Fettalkohol von 1-6 Kohlenstoffatomen eingesetzt.

Die rohe 4-Chlorbenzolsulfochlorid-Schmelze kann auch zur Herstellung von 4,4'-Dichlordiphenylsulfon nach Friedel-Crafts eingesetzt werden, indem man die genannte Schmelze mit katalytischen Mengen Eisen(III)-chlorid versetzt und dann auf 120-160°C erhitzt. Anschliessend tropft man eine mindestens stöchiometrische Menge Chlorbenzol zu, wobei Chlorwasserstoff entweicht. Nach Beendigung der Gasentwicklung wird ggf. überschüssiges Chlorbenzol mit Wasserdampf abdestilliert und das dabei angefallene 4,4'-Dichlordiphenylsulfon (im Gemisch mit wenig 4,2'-Isomerem) nach Abkühlen durch Filtration isoliert.

Die nachfolgenden Beispiele sollen die Erfindung näher erläutern, ohne sie darauf zu beschränken.

*Beispiel 1* (4-Chlorbenzolsulfochlorid)

Zu einer Mischung aus 122,3 Teilen Chlorsulfonsäure, 148,75 Teilen Thionylchlorid und 2,09 Teilen Dimethylformamid werden bei 80°C innerhalb von 2 h unter Rühren gleichmässig 112,5 Teile Chlorbenzol getropft. Die dabei entweichenden Abgase werden in einer zweistufigen Absorptionsanlage (Stufe 1 enthält 300 Teile Wasser, Stufe 2 406 Teile 12,8%ige wässerige Natronlauge) absorbiert. Nach beendeter Chlorbenzolzugabe wird 30 min bei 80°C nachgerührt. Die Absorptionsanlage enthält dann in der ersten Stufe ca. 400 g 20%ige Salzsäure, in der zweiten Stufe ca. 475 g 23%ige $NaHSO_3$-Lösung, die bei Folgereaktionen wieder eingesetzt werden können (vgl. hierzu Beispiel 17).

Die erhaltene rohe 4-Chlorbenzolsulfochlorid-Schmelze (ca. 213 Teile, Fp. ca. 45°C), die als Verunreinigungen noch ca. 4% 4,4'-Dichlordiphenylsulfon und 1% Dimethylformamid enthält, wird im Vakuum (15 mbar/142°C) ohne Kolonne überdestilliert.

Man erhält 196 Teile 4-Chlorbenzolsulfochlorid (Reinheitsgehalt laut Gaschromatographie 99%) vom Schmelzpunkt 53,5°C, was einer Ausbeute von 92,9 der Theorie, bezogen auf eingesetztes Chlorbenzol, entspricht. Verwendet man anstelle von Dimethylformamid eine aliquote Menge an N-Methylpyrrolidon oder Tetramethylharnstoff als Katalysator, so erhält man das 4-Chlorbenzolsulfochlorid in vergleichbaren Ausbeuten und Qualitäten.

*Beispiel 2* (4-Chlorbenzolsulfochlorid)

Arbeitet man gemäss Beispiel 1, lässt aber das als Katalysator fungierende Dimethylformamid weg, so muss die Zutropfzeit des Chlorbenzols auf 8 h und die Nachrührzeit auf 4 h erhöht werden, um Ausbeute und Qualität des destillierten 4-Chlorbenzolsulfochlorids reproduzieren zu können.

*Beispiele 3-7* (4-Chlorbenzolsulfochlorid)

Unter Verwendung der in der nachstehenden Tabelle angegebenen Molmengen Chlorsulfonsäure und Thionylchlorid werden bei den dort genannten Temperaturen und Zutropf-/Nachrührzeiten nach dem Verfahren des Beispiels 1 ebenfalls vollständig umgesetzte, weder Chlorsulfonsäure, noch Thionylchlorid enthaltende Schmelzen von rohem 4-Chlorbenzolsulfochlorid erhalten, die nach Vakuumdestillation die in der Tabelle aufgeführten Ausbeuten an qualitativ identischem 4-Chlorbenzolsulfochlorid liefern.

Der Gehalt der Rohschmelze an 4,4'-Dichlordiphenylsulfon ist angegeben:

| Beispiel | $ClSO_3H$ (Mol) | $OSCl_2$ (Mol) | Reakt. Temperatur (°C) | Zutropf- | Nachrühr- | Ausbeute (% d.Th.) | Sulfon-gehalt (%) |
|---|---|---|---|---|---|---|---|
| | | | | Zeit (h) | | | |
| 3 | 1,0 | 1,10 | 60 | 4 | 1 | 95,8 | 2,9 |
| 4 | 1,0 | 1,05 | 55 | 5 | 1 | 96,0 | 2,8 |
| 5 | 1,0 | 1,00 | 50 | 8 | 2 | 96,4 | 2,5 |
| 6 | 1,0 | 1,15 | 65 | 3,5 | 1 | 95,1 | 3,2 |
| 7 | 1,05 | 1,20 | 70 | 3 | 1 | 93,9 | 3,6 |

*Beispiel 8* (4-Chlorbenzolsulfamid)

Eine ausgehend von 112,5 Teilen Chlorbenzol gemäss Beispielen 1-7 erhaltene rohe 4-Chlorbenzolsulfochlorid-Schmelze (ca. 210-215 Teile, Fp. ca. 45°C) wird undestilliert aus einem auf 50-60°C geheizten Tropftrichter innerhalb 2 h zu einer gerührten Mischung aus 717 Teilen Wasser und 394,4 Teilen 25%iger wässeriger Ammoniaklösung (≙ 96 Teile NaOH) zulaufen und rührt 30 min unter Abkühlen auf 30-35°C nach. Durch Klärfiltration wird das in der Ausgangsschmelze enthaltene, jetzt ungelöste 4,4'-Dichlordiphenylsulfon abgetrennt und das wasserklare, farblose Filtrat mit 666 Teilen 30%iger Salzsäure (≙ 200 Teile HCl) auf pH 5-6 gestellt. Dabei wird die Temperatur durch Kühlen auf 20 bis 25°C gehalten. Man saugt ab, wäscht mit Wasser, trocknet und erhält 174 Teile 4-Chlorbenzolsulfamid vom Schmelzpunkt 146°C, was einer Ausbeute von 90,9% der Theorie, bezogen auf eingesetztes Chlorbenzol, entspricht. Das Produkt ist analysenrein. Der getrocknete Klärfilterrückstand (6-7 Teile) ist einheitliches 4,4'-Dichlordiphenylsulfon vom Schmelzpunkt 148°C (Die Ausbeute beträgt 4,2 bis 4,9% der Theorie, bezogen auf eingesetztes Chlorbenzol).

*Beispiel 9* (4-Chlorbenzolsulfanilid)

Ersetzt man im Beispiel 8 die angegebene Ammoniak-Menge durch 97,6 Teile Anilin, erhöht dabei die Zutropftemperatur auf 80-85°C, hält während des Zutropfens der rohen 4-Chlorbenzolsulfochlorid-Schmelze den pH-Wert durch gleichzeitigen Zulauf von 233 Teilen 25%iger Sodalösung (≙ 58,3 Teile $Na_2CO_3$) bei 6-8, und arbeitet im übrigen nach dem dort angegebenen Verfahren, so erhält man 246 Teile 4-Chlorbenzolsulfanilid vom Schmelzpunkt 104°C, was einer Ausbeute von 92% der Theorie, bezogen auf eingesetztes Chlorbenzol, entspricht.

*Beispiele 10-16:*

Nach den Verfahrensweisen der Beispiele 8 oder 9 werden die in nachfolgender Tabelle aufgeführten 4-Chlorbenzolsulfamide in gleicher Weise erhalten, wenn man die nach den Beispielen 1-7 aus 112,5 Teilen Chlorbenzol hergestellte rohe 4-Chlorbenzolsulfochlorid-Schmelze undestilliert mit den angegebenen Molmengen der angegebenen Amine $R-NH_2$ umsetzt. Ausbeuten und Schmelzpunkte sind ebenfalls in der Tabelle angegeben.

Tabelle

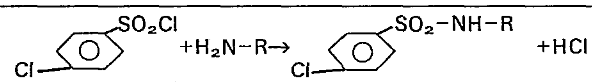

| Beispiel | R | Mol $R-NH_2$ | Ausbeute (%) | Schmelz-punkt (°C) |
|---|---|---|---|---|
| 10 | $-NH_2$ | 1,05 | 89,8 | 114 |
| 11 | $-CH_3$ | 1,80 | 91,6 | 64 |
| 12 | 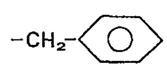 | 1,05 | 94,0 | 109 |
| 13 | 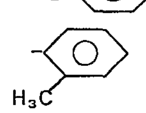 | 1,025 | 93,8 | 112 |
| 14 | 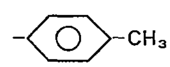 | 1,025 | 94,1 | 88 |
| 15 | 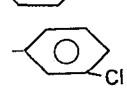 | 1,025 | 94,0 | 109 |
| 16 | 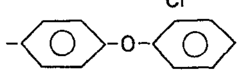 | 1,01 | 93,5 | 138 |

*Beispiel 17* (4-Chlorbenzolsulfinsäure)

Zu einer Mischung aus 475 Teilen 23%iger Natriumbisulfitlösung (≙ 108,2 Teile $NaHSO_3$) (vorteilhaft wird der Zwangsanfall aus der Abgasabsorption der 4-Chlorbenzolsulfochlorid-Herstellung gemäss Beispielen 1-7 verwendet), 131,8 Teilen 33%iger Natronlauge (43,6 Teile NaOH), 8,3 Teilen 85%iger Phosphorsäure und 1200 Teilen Wasser, die einen pH-Wert von ca. 7,5 aufweist, wird in 2 h unter Rühren bei 60° C die ausgehend von 112,5 Teilen Chlorbenzol gemäss beispielen 1-7 erhaltene rohe, undestillierte 4-Chlorbenzolsulfochlorid-Schmelze (ca. 210-215 Teile, Fp. ca. 45° C) getropft. Durch gleichzeitigen Zulauf von 236 Teilen 33%iger Natronlauge (≙ 78,7 Teile NaOH) wird der pH-Wert bei 7,5-8,0 gehalten. Man rührt 30 min nach, klärt bei 60-70° C vom ungelösten 4,4'-Dichlordiphenylsulfon (nach dem Trocknen 6-7 Teile analysenreines Produkt, Fp. 148° C), stellt das Filtrat mit ca. 200 Teilen 30%iger Salzsäure (≙ 60 Teile HCl) sauer und isoliert die beim Abkühlen ausfallende 4-Chlorbenzolsulfinsäure durch Filtration. Nach Waschen mit Eiswasser und Trocknen erhält man 145 Teile 4-Chlorbenzolsulfinsäure vom Schmelzpunkt 99° C (82,2% der Theorie, bezogen auf eingesetztes Chlorbenzol).

*Beispiel 18* (4-Chlorphenyl-methylsulfon)

Die gemäss Beispiel 17 erhaltene, durch Klärfiltration bei 60-70° C vom 4,4'-Dichlordiphenylsulfon befreite wässerige Lösung des 4-Chlorbenzolsulfinats (pH-Wert 7,5-8,0) wird bei dieser Temperatur unter Rühren innerhalb 60 min mit 160 Teilen Dimethylsulfat versetzt, 1 h bei 60° C nachgerührt, evtl. vorhandenes überschüssiges Dimethylsulfat durch Zugabe von 20 Teilen 25%igem wässerigen Ammoniak zerstört und der ausgefallene Niederschlag nach Abkühlen durch Filtration isoliert. Nach Waschen mit Wasser und Trocknen bei 60° C im Vakuum erhält man 170 Teile 4-Chlorphenyl-methylsulfon vom Schmelzpunkt 96° C (89,2% der Theorie, bezogen auf eingesetztes Chlorbenzol).

Verwendet man an Stelle des Dimethylsulfats eine aliquote Menge an Methylchlorid, führt aber die Alkylierungsreaktion unter Druck bei 95° C durch und arbeitet im übrigen in der angegebenen Weise, so erhält man das 4-Chlorphenylmethylsulfon in vergleichbarer Ausbeute und Qualität.

*Beispiel 19* (4-Chlorphenyl-β-oxethylsulfon)

Die gemäss Beispiel 17 erhaltene, durch Klärfiltration bei 60 bis 70° C vom 4,4'-Dichlordiphenylsulfon befreite wässerige Lösung des 4-Chlorbenzolsulfinats (pH-Wert 7,5-8,0) wird bei dieser Temperatur unter Rühren in 1-2 h gleichmässig mit ca. 129 Teilen Ethylenoxid begast, wobei der pH-Wert durch gleichzeitige Zugabe von 530 Teilen 20%iger Phosphorsäure (=106 Teilen $H_3PO_4$) konstant bei 6,5-7,0 gehalten wird. Man rührt 5 h bei 60° C und konstantem pH von 6,5-7,0 nach, führt bei dieser Temperatur eine Klärfiltration durch und kühlt das Filtrat auf 10° C. Das dabei sich flüssig abscheidende 4-Chlorphenyl-β-oxethylsulfon wird abgetrennt. Durch zweimaliges Ausrühren der wässerigen Phase mit Methylisobutylketon, jeweilige Phasentrennung und Abdestillieren des Extraktionsmittels aus den mit der flüssig abgetrennten 4-Chlorphenyl-β-oxethylsulfonschmelze vereinigten Extrakten erhält man 191 Teile 92%iges 4-Chlorphenyl-β-oxethylsulfon (79,7 der Theorie, bezogen auf eingesetztes Chlorbenzol). Das Produkt kristallisiert nur zögernd und weist keinen scharfen Schmelzpunkt auf.

*Beispiel 20* (4-Chlorbenzolsulfonsäureethylester)

Eine ausgehend von 112,5 Teilen Chlorbenzol gemäss Beispielen 1-7 erhaltene rohe 4-Chlorbenzolsulfochlorid-Schmelze wird undestilliert innerhalb 2 h bei 50-60° C mit 69 Teilen Ethanol versetzt. Man rührt bis zum Ende der sofort einsetzenden HCl-Entwicklung (ca. 60 min) nach, destilliert das überschüssig eingesetzte Ethanol bei Normaldruck ab und fraktioniert anschliessend im Vakuum. Bei 172° C/15 mbar geht der 4-Chlorbenzolsulfonsäureethylester rein über. Man erhält 204 Teile vom Schmelzpunkt 27° C, was einer Ausbeute von 92,5% der Theorie bezogen auf eingesetztes Chlorbenzol, entspricht.

*Beispiel 21* (4-Chlorbenzolsulfonsäuremethylester)

Zu 96 Teilen Methanol von 20° C wird aus einem auf 50-60° C geheizten Tropftrichter innerhalb 2 h unter Rühren die gemäss Beispielen 1-7 aus 112,5 Teilen Chlorbenzol hergestellte rohe, undestillierte 4-Chlorbenzolsulfochlorid-Schmelze getropft. Anschliessend heizt man in 1 h auf 60-70° C, destilliert das überschüssige Methanol bei Normaldruck ab und fraktioniert den verbleibenden Sumpf im Vakuum. Bei 154° C/8 mbar gehen 192 Teile reiner 4-Chlorbenzolsulfonsäuremethylester (Schmelzpunkt 51° C) über, was einer Ausbeute von 93,0% der Theorie, bezogen auf eingesetztes Chlorbenzol, entspricht.

*Beispiel 22* (4,4'-Dichlordiphenylsulfon)

Eine ausgehend von 112,5 Teilen Chlorbenzol gemäss Beispielen 1-7 erhaltene rohe 4-Chlorbenzolsulfochlorid-Schmelze wird mit 550 Teilen Chlorbenzol verdünnt. Man setzt bei ca. 30° C 10 Teile Eisen(III)-chlorid zu und erhitzt anschliessend unter Rühren zum Rückfluss. Nach 12 h wird das überschüssige Chlorbenzol mit Wasserdampf abgeblasen und aus der zurückbleibenden wässerigen Suspension das kristalline, farblose Dichlordiphenylsulfon durch Filtration bei 20-30° C abgetrennt. Nach Waschen mit Wasser und Trocknen erhält man 275 Teile eines Isomerengemisches, das aus 93,1% 4,4'-Dichlordiphenylsulfon und 6,9% 4,2'-Dichlordiphenylsulfon (Analyse durch Gaschromatographie) besteht.

Man erhält auf diese Weise 95,8% der Theorie Dichlordiphenylsulfone, bezogen auf umgesetztes Chlorbenzol. Der Gehalt am erwünschten 4,4'-Isomeren ist dem aus anderen Synthesen erzielbaren vergleichbar.

## Patentansprüche

1. Verfahren zur Herstellung von 4-Chlorphenylsulfonylverbindungen der allgemeinen Formel (1)

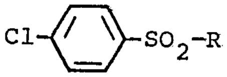

(1)

in welcher R ein Wasserstoff- oder Chloratom oder eine Alkyl-, Oxalkyl- oder Alkoxygruppe, eine Hydrazinogruppe, eine primäre Aminogruppe, primäre oder sekundäre Alkylaminogruppe, Aralkylaminogruppe oder Phenylaminogruppe bedeutet, wobei die Phenylgruppe durch Chlor- oder Bromatome, Alkylgruppen, Alkoxygruppen oder Phenoxygruppen substituiert sein kann, oder eine 4-Chlorphenylgruppe bedeutet, durch Reaktion von Chlorbenzol mit Chlorsulfonsäure und Thionylchlorid zum 4-Chlorbenzolsulfochlorid und dessen Umsetzung mit Ammoniak, Hydrazin, primärem oder sekundärem aliphatischem Amin, aromatischem Amin, Alkalimetallsulfit oder Alkanol zu Verbindungen der genannten Formel (1), dadurch gekennzeichnet, dass man 1 Mol Chlorbenzol langsam mit einem Gemisch aus 1,0 bis 1,05 Mol Chlorsulfonsäure und 1,0 bis 1,25 Mol Thionylchlorid bei Temperaturen von 20 bis 90°C mit der Massgabe, dass das Reaktionsgemisch stets flüssig ist, ggfs. in Gegenwart eines für die Sulfonsäurechloridbildung mittels Thionylchlorid an sich bekannten Katalysators, umsetzt und das hierbei in Form einer Schmelze angefallene 4-Chlorbenzolsulfochlorid vorzugsweise direkt oder nach Abtrennung von dem als Nebenprodukt angefallenen 4,4'-Dichlordiphenylsulfon umsetzt

a) mit einer wässerigen Lösung, Suspension oder Emulsion von Ammoniak, Hydrazin, eines primären oder sekundären aliphatischen Amins oder aromatischen Amins der Benzolreihe bei Temperaturen von 0 bis 100°C zu den entsprechenden 4-Chlorbenzolsulfonamiden, oder

b) mit einer wässerigen Alkalimetallsulfitlösung bei Temperaturen von 20 bis 90°C bei pH-Werten von 6 bis 9 zu den entsprechenden 4-Chlorbenzolsulfinaten und daraus durch Neutralisieren der wässerigen Lösung mittels Säure zu der freien 4-Chlorbenzolsulfinsäure oder durch Umsetzen mit Alkylierungsmitteln zu den entsprechenden 4-Chlorphenylalkylsulfonen, oder

c) mit der mindestens stöchiometrischen Menge eines Alkanols bei Temperaturen von 20 bis 90°C zu den entsprechenden 4-Chlorbenzolsulfonsäurealkylestern oder

d) mit der mindestens stöchiometrischen Menge Chlorbenzol in Gegenwart katalytischer Mengen Eisen(III)-chlorid bei Temperaturen von 120-160°C zum 4,4'-Dichlordiphenylsulfon.

2. Verfahren nach Anspruch 1, dadurch gekennzeichnet, dass man das Chlorbenzol gleichmässig im Verlauf von 2 bis 8 h der vorgelegten Mischung aus Chlorsulfonsäure und Thionylchlorid zugibt.

3. Verfahren nach Ansprüchen 1 oder 2, dadurch gekennzeichnet, dass man die Sulfochlorierung des Chlorbenzols in Gegenwart eines für die Sulfonsäurechloridbildung mittels Thionylchlorid an sich bekannten Katalysators durchführt.

4. Verfahren nach Anspruch 3, dadurch gekennzeichnet, dass man als Katalysator Dimethylformamid, Dimethylacetamid, N-Methylpyrrolidon, N-Methylacetamid, Tetramethylharnstoff oder Triäthylamin in einer Menge von 0,5-5 Molprozent, bezogen auf 1 Mol Chlorbenzol, einsetzt.

5. Verfahren nach Anspruch 1, dadurch gekennzeichnet, dass man eine wässerige Lösung, Suspension oder Emulsion von Ammoniak, Hydrazin, primärem oder sekundärem aliphatischem Amin oder aromatischem Amin bei Temperaturen zwischen 0 und 100°C innerhalb von 1 bis 5 h mit der rohen 4-Chlorbenzolsulfochlorid-Schmelze versetzt und die gebildeten 4-Chlorbenzolsulfonamide, ggf. nach Zusatz stöchiometrischer Mengen an Alkalihydroxyd, in Form der freien wasserlöslichen Amide bzw. ihrer wasserlöslichen Alkalisalze in Lösung hält bzw. bringt und nach Abfiltrieren vom ungelösten 4,4'-Dichlordiphenylsulfon die 4-Chlorbenzolsulfonamide in bekannter Weise ausfällt und isoliert.

6. Verfahren nach Anspruch 1, dadurch gekennzeichnet, dass man eine wässrige Alkalimetallsulfitlösung bei Temperaturen von 20 bis 90°C innerhalb von 1 bis 5 h gleichzeitig mit der rohen 4-Chlorbenzolsulfochlorid-Schmelze und Alkalilauge bei Einhalten eines pH-Wertes von 6-9 versetzt, die erhaltene wässerige Lösung von gebildetem 4-Chlorbenzolsulfinat vom ungelösten 4,4'-Dichlordiphenylsulfonabfiltriert und aus dem wässerigen Filtrat das 4-Chlorbenzolsulfinat bzw. die freie 4-Chlorbenzolsulfinsäure in bekannter Weise isoliert.

7. Verfahren nach Anspruch 1, dadurch gekennzeichnet, dass man das in Form einer wässerigen Lösung angefallene 4-Chlorbenzolsulfinat ohne Isolierung mit einem Dialkylsulfat, Alkylhalogenid oder Alkylenoxid bei Temperaturen von 20-150°C, ggf. unter Druck, umsetzt, wobei bei Verwendung eines Alkylenoxids durch gleichzeitige Zugabe von Mineralsäure ein pH-Wert von 6-8 eingehalten wird.

## Claims

1. A process for the preparation of 4-chlorophenylsulfonyl compounds of the general formula (1)

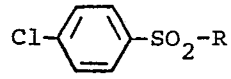

(1)

wherein

R represents a hydrogen or chlorine atom or an alkyl, oxalkyl or alkoxy group, a hydrazino group, a primary amino, a primary or secondary alkylamino, an aralkylamino or a phenylamino group, the phenyl group being optionally substituted by chlorine or bromine atoms, alkyl, alkoxy or phenoxy groups, or R represents a 4-chlorophenyl group, by reacting chlorobenzene with chlorosulfonic acid and thionyl chloride to give 4-chlorobenzenesulfochloride followed by a reaction of the latter with ammonia, hydrazine, primary or secondary aliphatic amine, aromatic amine, alkali metal sulfite or alkanol to give compounds of said formula (1), which comprises slowly reacting 1 mol of chlorobenzene with a mixture of 1.0 mol to 1.05 mol of chlorosulfonic acid and 1.0 mol to 1.25 mol of thionyl chloride at a temperature of about 20 to 90°C, provided that the reaction mixture always remains liquid, optionally in the presence of a catalyst commonly used for the formation of sulfonic acid chloride by means of thionyl chloride, and subsequently reacting 4-chloro-benzene-sulfochloride thus obtained as a melt, preferably directly or after its separation from 4,4'-dichlorodiphenylsulfone obtained as a byproduct,

a) with an aqueous solution, suspension or emulsion of ammonia, hydrazine, a primary or secondary aliphatic amine or an aromatic amine of the benzene series, at a temperature of about 0 to about 100°C to give the corresponding 4-chlorobenzenesulfonamides, or

b) with an aqueous alkali metal sulfite solution, at a temperature of about 20 to about 90°C at a pH-value of 6 to 9 to give the corresponding 4-chlorobenzenesulfinates followed by neutralization of the aqueous solution by an acid to give the free 4-chlorobenzenesulfinic acid or by a reaction with alkylation agents to give the corresponding 4-chlorophenylalkylsulfones, or

c) with the at least stoichiometric quantity of an alkanol, at a temperature of about 20 to about 90°C to give the corresponding 4-chlorobenzenesulfonic acid alkyl ester, or

d) with the at least stoichiometric quantity of chlorobenzene in the presence of catalytic amounts of iron(III)-chloride at temperatures of from 120 to 160°C to give 4,4'-dichlorodiphenylsulfone.

2. The process as claimed in claim 1, which comprises uniformly adding chlorobenzene within 2 to 8 hours to the previously introduced mixture of chlorosulfonic acid and thionyl chloride.

3. The process as claimed in claim 1 or 2, which comprises carrying out the sulfonation of the chlorobenzene in the presence of a catalyst known for use for the formation of sulfonic acid chloride by means of thionyl chloride.

4. The process as claimed in claim 3, which comprises using as the catalyst dimethyl formamide, dimethyl acetamide, N-methyl pyrrolidone, N-methyl acetamide, tetramethyl urea or triethylamine in an amount of from 0.5 to 5% by mol, referred to 1 mol of chlorobenzene.

5. The process as claimed in claim 1, which comprises adding the crude 4-chlorobenzenesulfochloride melt to an aqueous solution, suspension or emulsion of ammonia, hydrazine, primary or secondary aliphatic amine or aromatic amine at a temperature of from 0 to 100°C within 1 to 5 hours and keeping in dissolved state or bringing to dissolution the 4-chlorobenzenesulfonamides formed, in the form of the free water-soluble amides or of their water-soluble alkali metal salts, optionally on addition of a stoichiometric quantity of alkali metal hydroxyde and, after filtration from the undissolved 4,4'-dichlorodiphenylsulfone, precipitating and isolating the 4-chlorobenzenesulfonamides in a known manner.

6. The process as claimed in claim 1, which comprises simultaneously adding the crude 4-chlorobenzenesulfochloride melt and the alkali metal hydroxide solution to an aqueous alkali metal sulfite solution at temperatures of from 20 to 90°C within 1 to 5 hours, while maintaining a pH of 6-9, separating the aqueous solution of 4-chlorobenzenesulfinate obtained from undissolved 4,4'-dichlorodiphenylsulfone by filtration and isolating 4-chlorobenzenesulfinate or the free 4-chlorbenzenesulfinic acid from the aqueous filtrate in a known manner.

7. The process as claimed in claim 1, which comprises reacting the 4-chlorobenzenesulfinate obtained in the form of an aqueous solution without isolation with a dialkyl sulfate, an alkyl halide or an alkylene oxide at temperatures from 20 to 150°C, optionally under pressure, a pH-value of 6-8 being maintained by simultaneous addition of a mineral acid, if alkylene oxide is used.

## Revendications

1. Procédé de préparation de composés chloro-4-phénylsulfoniques répondant à la formule générale 1 :

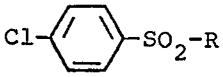

(1)

dans laquelle R représente un atome d'hydrogène ou de chlore, un radical alkyle, hydroxyalkyle ou alcoxy, un radical hydrazino, un radical amino non substitué, un radical alkylamino primaire ou secondaire, un radical aralkylamino ou un radical phénylamino, le radical phényle pouvant porter, comme substituants, des atomes de chlore ou de brome, des radicaux alkyles, des radicaux alcoxy ou des radicaux phénoxy, ou R représente un radical chloro-4 phényle, par réaction du chlorobenzène avec l'acide chlorosulfonique et le chlorure de thionyle pour obtenir le chlorure de chloro-4 benzènesulfonyle, et réaction de celui-ci avec l'ammoniac, l'hydrazine, une amine aliphatique primaire ou secondaire, une amine aromatique, un sulfite de métal alcalin ou un alcanol, pour obtenir des composés répondant à la formule 1 précédente, procédé caractérisé en ce qu'on fait réagir 1 mol de chlorobenzène, lentement, avec un mélange constitué de 1,0 à 1,05 mol d'acide chlorosulfonique et de 1,0 à 1,25 mol de chlorure de thionyle, à des températures de 20 à 90°C, avec la condition que le mélange réactionnel soit toujours liquide, éventuellement en présence d'un catalyseur connu pour la formation de composés chlorosulfonyliques au moyen du chlorure de thionyle, et on fait réagir le chlorure de chloro-4-benzènesulfonyle qui a été ainsi obtenu sous la forme d'un produit fondu, de préférence directement ou après séparation de la dichloro-4,4' diphénylsulfone formée comme produit secondaire,

a) avec une solution, suspension ou émulsion aqueuse d'ammoniac, d'hydrazine, d'une amine aliphatique primaire ou secondaire ou d'une amine aromatique de la série benzénique, à des températures de 0 à 100°C, réaction qui conduit aux chloro-4 benzènesulfonamides correspondants, ou

b) avec une solution aqueuse d'un sulfite de métal alcalin, à des températures de 20 à 90°C et à des pH de 6 à 9, de manière à obtenir les chloro-4 benzènesulfinates correspondants, à partir desquels on obtient, par neutralisation de la solution aqueuse au moyen d'un acide, l'acide chloro-4 benzènesulfinique libre, ou, par réaction avec des agents d'alkylation, les (chloro-4-phényl)alkylsulfones correspondantes, ou

c) avec une quantité au moins égale à la quantité stœchiométrique d'un alcool, à des températures de 20 à 90°C, pour obtenir les chloro-4-benzènesulfonates d'alkyles correspondants, ou

d) avec une quantité de chlorobenzène au moins égale à la quantité stœchiométrique, en présence de quantités catalytiques de chlorure de fer-(III), à des températures de 120 à 160°C, réaction qui conduit à la dichloro-4,4'-diphénylsulfone.

2. Procédé selon la revendication 1, caractérisé en ce qu'on ajoute le chlorobenzène régulièrement, en 2 à 8 h, au mélange, préalablement mis en place, d'acide chlorosulfonique et de chlorure de thionyle.

3. Procédé selon l'une des revendications 1 et 2, caractérisé en ce qu'on effectue la sulfochloration du chlorobenzène en présence d'un catalyseur connu pour la formation de sulfochlorures au moyen du chlorure de thionyle.

4. Procédé selon la revendication 3, caractérisé en ce qu'on utilise, comme catalyseur, le diméthylformamide, le diméthylacétamide, la N-méthylpyrrolidone, le N-méthylacétamide, la tétraméthylurée ou la triéthylamine en une quantité de 0,5 à 5% en moles par rapport au chlorobenzène.

5. Procédé selon la revendication 1, caractérisé en ce qu'on ajoute en 1 à 5 h la masse fondue brute de chlorure de chloro-4 benzènesulfonyle à une solution, suspension ou émulsion aqueuse d'ammoniac, d'hydrazine, d'une amine aliphatique primaire ou secondaire ou d'une amine aromatique, à des températures comprises entre 0 et 100°C, on maintient en solution ou on fait passer en solution les chloro-4 benzènesulfonamides formés, éventuellement après y avoir ajouté des quantités stœchiométriques d'un hydroxyde de métal alcalin, sous la forme des amides libres solubles dans l'eau ou sous la forme de leurs sels de métaux alcalins solubles dans l'eau, et, après avoir séparé par filtration la dichloro-4,4'-diphénylsulfone non dissoute, on fait précipiter et on isole de manière connue les chloro-4-benzènesulfonamides.

6. Procédé selon la revendication 1, caractérisé en ce qu'on ajoute en 1 à 5 h, tout en maintenant le pH à 6-9, en même temps la masse fondue brute de chlorure de chloro-4-benzènesulfonyle et la solution d'hydroxyde de métal alcalin à une solution aqueuse d'un sulfite de métal alcalin, à des températures de 20 à 90°C, on élimine la dichloro-4,4'-diphénylsulfone non dissoute, par filtration, de la solution aqueuse obtenue du chloro-4-benzènesulfinate formé et, du filtrat aqueux, on isole de manière connue le chloro-4-benzènesulfinate ou l'acide chloro-4-benzènesulfinique libre.

7. Procédé selon la revendication 1, caractérisé en ce qu'on fait réagir le chloro-4-benzènesulfinate obtenu sous la forme d'une solution aqueuse, sans l'isoler, avec un sulfate de dialkyle, un halogénure d'alkyle ou un oxyde d'alkylène, à des températures de 20 à 150°C, éventuellement sous pression, et, dans le cas où on utilise un oxyde d'alkylène, on maintient le pH à 6-8 en ajoutant en même temps un acide minéral.